# EUROPÄISCHE PATENTSCHRIFT

(11) **EP 0 578 146 B1**
(45) Veröffentlichungstag und Bekanntmachung des Hinweises auf die Patenterteilung: **16.08.1995**
(21) Anmeldenummer: 93110577.9
(22) Anmeldetag: 02.07.1993
(51) Int. Cl.: G01N 1/26, B08B 9/46, G01N 1/00, B08B 101/08

(54) **Verfahren und Vorrichtung zum Prüfen von Flaschen auf das Vorhandensein von Verunreinigungen**
Method and device for testing of bottles for contamination
Procédé et dispositif pour détecter la présence de salissures dans des bouteilles

(30) Priorität: 09.07.1992 CH 2166/92; 04.03.1993 CH 656/93; 11.02.1993 CH 415/93; 11.02.1993 CH 416/93
(43) Veröffentlichungstag der Anmeldung: 12.01.1994
(73) Patentinhaber: ELPATRONIC AG, CH-6303 Zug (CH)
(72) Erfinder: Gysi, Peter, CH-5454 Bellikon (CH); Mueller, Martin, Dr., CH-8918 Unterlunkhofen (CH); Van der Schaar, Felix, CH-8472 Seuzach (CH); Huesser, Theo, CH-8964 Rudolfstetten (CH); Robertson, Peter Murday, CH-8185 Winkel (CH); Zumbach, Melchior, Dr., CH-8600 Dübendorf (CH)

(56) Entgegenhaltungen:
- DE-A- 1 598 415
- DE-A- 3 621 976
- DE-A- 3 624 419
- DE-U- 8 806 138
- FR-A- 1 344 848
- US-A- 3 321 954
- US-A- 5 116 764

## Beschreibung

Die Erfindung betrifft ein Verfahren zum Prüfen von zur erneuten Befüllung rücklaufenden Mehrwegflaschen, insbesondere PET-Flaschen, auf das Vorhandensein von Verunreinigungen, durch Prüfung von jeweils jeder Flasche entnommenem Gas, wobei die Flaschen entlang eines Förderweges transportiert werden. Ferner betrifft die Erfindung eine Vorrichtung zur Durchführung des Verfahrens.

Bei Mehrwegflaschen, insbesondere bei Kunststoff-Flaschen, wie z.B. PET-Flaschen, welche nicht bei hohen Temperaturen gewaschen werden können, stellt sich das Problem, das Verunreinigungen sicher erkannt werden müssen, damit verunreinigte Flaschen ausgeschieden, bzw. der erneuten Befüllung entzogen werden können. Insbesondere müssen dabei zurückgegebene Flaschen erkannt werden können, die ein Benützer für potentiell gefährliche Stoffe (Gifte, Lösungsmittel) verwendet hat. Es ist bereits bekannt, zu diesem Zweck jeweils eine Gasprobe aus der Flasche zu entnehmen und diese durch Flammen-Ionisations-Detektion (US-A-3321954) oder durch Photo-Ionisations-Detektion (PID) zu untersuchen. Damit kann das Vorhandensein auch geringer Spuren unerwünschter Stoffe in der Flasche bzw. im Kunststoff-Material erkannt werden. Da solche Prüfvorrichtungen einen hohen Flaschendurchsatz pro Minute (erwünscht z.B. 250 bis 300 Flaschen pro Min.) aufweisen müssen, damit eine derartige Prüfung in einem industriellen Abfüllbetrieb vorgenommen werden kann, kamen bisher jeweils eine grosse Zahl einzelner PID-Geräte zum Einsatz, d.h. jeder von mehreren zu prüfenden Flaschen wurde ein eigenes PID-Gerät zugewiesen. Dies bedingt indes einen hohen Kosten-, Wartungs- und Kalibrieraufwand.

Es ist daher an sich wünschenswert, die Anzahl der Prüfgeräte zu verringern. Damit der Flaschendurchsatz nicht vermindert wird, ist es dabei allerdings erforderlich, dass die Messzeit pro Flasche verkürzt wird, da bei verringerter Anzahl Prüfgeräte jedes Prüfgerät in der gleichen Zeitdauer mehr als eine Flasche prüfen muss. Es ist dabei aber insbesondere zu beachten, dass nicht nur die eigentliche Messdauer für die jeweilige Gasprobe im Prüfgerät massgeblich ist, sondern auch eine Erholungszeit einzuberechnen ist, welche das Prüfgerät benötigt, um nach der Feststellung einer starken Verunreinigung wieder genügend Empfindlichkeit für die Feststellung einer schwachen Verunreinigung aufzuweisen; es wird dafür auch der Begriff Memory-Effekt verwendet. Ein solcher Effekt tritt bei allen geeigneten Prüfgeräten für die Gasproben auf, so bei den bereits genannten PID-Prüfgeräten und auch bei Massenspektrometern.

Der Erfindung liegt deshalb die Aufgabe zugrunde, ein Prüfverfahren für Flaschen zu schaffen, bei welchem trotz hohem Flaschendurchsatz mit wenigen Prüfgeräten gearbeitet werden kann, bzw. bei welchem der Memory-Effekt der Prüfgeräte den Flaschendurchsatz nicht reduziert, wodurch die Flaschenprüfung mit einem geringeren apparativen Kosten-, Wartungs- und Kalibrieraufwand erfolgen kann.

Dies wird bei einem Verfahren der eingangs genannten Art dadurch erreicht, dass die Prüfung jeder Flasche durch mindestens zwei entlang des Förderweges nacheinander angeordnete Prüfstationen erfolgt, wobei die erste Prüfstation eine untere Ansprechschwelle für Verunreinigungen aufweist, welche ein Mehrfaches der unteren Erkennungsschwelle für Verunreinigungen der zweiten Prüfstation beträgt, und dass bei Flaschen, auf deren Verunreinigung die erste Prüfstation angesprochen hat, bei deren Durchlauf durch die zweite Prüfstation die zweite Prüfstation deaktiviert wird.

Gemäss einer alternativen Ausgestaltung des Verfahrens nach Anspruch 4 werden Flaschen, auf deren Verunreinigung die erste Prüfstation angesprochen hat, vor Erreichen der zweiten Prüfstation aus dem Förderweg ausgeschieden.

Die erste Prüfstation, die mit einer relativ hohen Ansprechschwelle für Verunreinigungen prüft, kann stark verunreinigte Flaschen erkennen, welche nachfolgend die zweite Prüfstation zwar durchlaufen, nicht aber in der zweiten, empfindlicheren Prüfstation geprüft werden. Alternativ werden diese Flaschen vorher ausgeschieden. Die zweite Prüfstation wird damit von stark verunreinigten Flaschen, die einen ausgeprägten Memory-Effekt bewirken würden, entlastet. Dies bedeutet, dass einerseits bei der zweiten Prüfstation eine tiefe Erkennungsschwelle für Verunreinigungen vorgesehen werden kann, was wünschbar ist, um auch geringe schädliche Verunreinigungen feststellen zu können, dass aber dennoch kein zeitkritischer Memory-Effekt auftreten kann, da die stark verunreinigten Flaschen, die dies bewirken würden, in der zweiten Prüfstation nicht mehr geprüft werden bzw. diese nicht durchlaufen. Der dynamische Bereich, der von der zweiten Prüfstation abgedeckt werden muss, ist also von oben her, d.h. von den starken Verunreinigungen her, durch die erste Prüfstation beschnitten, was bei der zweiten Prüfstation die genannten positiven Effekte ergibt. Bei der ersten Prüfstation ist der Memory-Effekt nicht kritisch, da hier nur starke Verunreinigungen erkannt werden müssen, was auch nach einer vorhergehenden starken Verunreinigung jeweils nach sehr kurzer Erholungszeit möglich ist. Hier ist also der dynamische Bereich von unten her, d.h. von den schwachen Verunreinigungen her, beschnitten, indem die erste Station nur auf starke Verunreinigungen anspricht. Es kann dabei durchaus eine erste Prüfstation eingesetzt sein, deren Erkennungsschwelle grundsätzlich ebenfalls tief wäre, die Ansprechempfindlichkeit, bei welcher die Prüfstation aktiv wird, wird aber relativ hoch gewählt.

Diese Abstimmung der Bereiche zwischen den Prüfstationen macht es bei einer bevorzugten Ausführungsart möglich, als zweite, vorteilhaft sehr empfindliche Prüfung eine massenspektrometrische Prüfung vorzusehen, deren ausgeprägter nachteiliger Memory-Effekt durch das erfindungsgemässe Verfahren gemäss beiden alternativen Ausführungen weitgehend eingeschränkt wird, so dass das Massenspektrometer rasch aufeinanderfolgende Flaschenmessungen durchführen kann und der hohe Flaschendurchsatz möglich ist. Als kostengünstige erste Prüfung wird dabei eine Prüfung mit Photo-Ionisations-Detektion verwendet. Bevorzugterweise erfolgt das Nichtprüfen der verunreinigten Flaschen derart, dass das Gasentnahmeorgan nicht in die Flasche eintaucht, um dessen Verunreinigung zu vermeiden. In einer weiteren bevorzugten Ausführungsart wird sogar ein Verschlusselement oberhalb der Flaschenöffnung wirksam, um das Ansaugen von Gas aus der verschmutzten Flasche sicher zu vermeiden.

Der Erfindung liegt ferner die Aufgabe zugrunde, eine Vorrichtung zur Durchführung des Verfahrens zu schaffen. Dies wird mit der Vorrichtung nach Anspruch 8 erreicht bzw. mit einer Vorrichtung nach Anspruch 12.

Nachfolgend werden Ausführungsbeispiele der Erfindung anhand der Zeichnungen näher erläutert. Dabei zeigt
Figur 1 schematisch eine Fördereinrichtung für Flaschen mit zwei Prüfstationen;
Figur 2 eine schematische Draufsicht auf eine spezielle Ausgestaltung der Fördereinrichtung, und
Figur 3 ein Ablaufschema der verschiedenen Prüfungen an jeder Flasche;
Figur 4 eine Seitenansicht des Hauptkarussells von Figur 2 und
Figur 5 eine Teilansicht des Verschlussmechanismus.

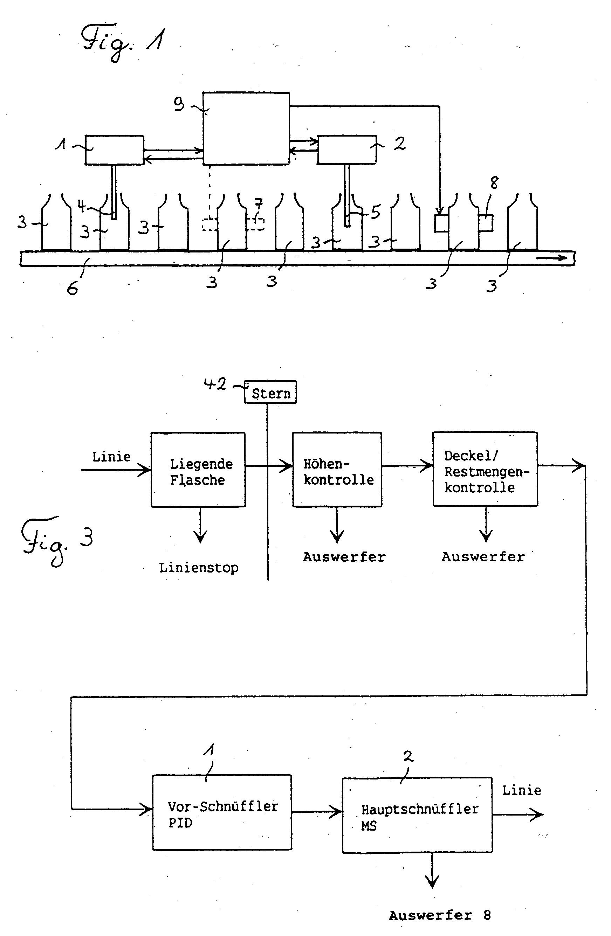

Figur 1 zeigt schematisch ein Föderband 6 auf dem Flaschen stehend gefördert werden. Bei den Flaschen handelt es sich in dem Beispiel um Mehrweg-PET-Flaschen, welche anschliessend an die nun beschriebene Prüfung einer Waschanlage zugeführt und danach erneut mit einem Getränk befüllt werden. Die erfindungsgemässe Prüfung verwendet zwei Prüfstationen 1, 2. Mit der Prüfstation 1 wird eine starke Verunreinigung der Flasche mit schädlichen Substanzen ermittelt, worunter z.B. solche Substanzen wie Benzin, Lösungsmittel, Lacke, Insektizide, Alkohol usw. verstanden werden. Durch die Prüfstation 1 wird jeweils eine Gasprobe aus der Flasche entnommen, was z.B. mittels einer ein- und ausfahrbaren Sonde 4 geschehen kann, wie dies schematisch gezeigt wird. Insbesondere bei der Station 1 für die starke Verunreinigung kann es aber auch genügen, wenn oberhalb der Flaschenöffnung Gas angesaugt wird. Auch das zusätzliche Einblasen von Luft in die Flasche ist möglich, um die Entnahme einer Gasprobe zu erleichtern. Die entnommene Gasprobe wird analysiert, was vorzugsweise auf bekannte Weise mittels Photo-Ionisations-Detektion erfolgt. Ein geeignetes Gerät ist z.B. der Photo-Ionisations-Detektor Modell PI 52-02A der Firma HNU Systems, Inc. Die Ansprechschwelle wird dabei so eingestellt, dass stark verunreinigte Flaschen sicher erkannt werden. Die Information, ob eine Flasche derart verunreinigt ist, wird an eine Steuereinrichtung 9 abgegeben. Diese steuert in Abhängigkeit vom Signal der Prüfstation 1 die Prüfstation 2 so, dass diese die als verschmutzt erkannten Flaschen nicht prüft. Alternativ steuert die Steuereinrichtung 9 in Abhängigkeit von der Prüfstation 1 einen strichliniert dargestellten Auswerfer 7, welcher in Förderrichtung nach der Prüfstation 1 angeordnet ist, und welcher die als kontaminiert erkannte Flasche aus der Fördereinrichtung auswirft oder auf eine andere Förderbahn auswirft, bevor diese die Prüfstation 2 erreicht hat. Die Prüfstation 2 prüft die von der Station 1 als sauber betrachteten Flaschen erneut auf das Vorhandensein schädlicher Substanzen. Hierbei muss aber nun nicht mehr mit sehr starken Konzentrationen schädlicher Stoffe in der Gasprobe gerechnet werden, weshalb eine tiefe Erkennungsschwelle gewählt werden kann, ohne dass befürchtet werden muss, dass zwischen den Messungen eine lange Zeit zum Abbau des Memory-Effektes nötig ist. Es kann also trotz hoher Empfindlichkeit schnell hintereinander gemessen werden, was einen hohen Flaschendurchsatz erlaubt. Zur Messung wird hierbei ebenfalls eine Gasprobe aus der Flasche mittels einer ein- und ausfahrbaren Sonde 5 entnommen. Vorzugsweise erfolgt hier die Messung mit einem handelsüblichen Massenspektrometer, was eine besonders genaue Analyse auch geringer Kontaminationen ermöglicht und deshalb die eindeutige Entscheidung, ob die Flasche noch brauchbar ist oder nicht. Unbrauchbare Flaschen werden von einem weiteren Auswerfer 8 in Abhängigkeit von der Analyse aus dem Förderweg ausgestossen oder auf eine andere Förderbahn ausgelenkt. Anstelle des Massenspektrometers kann aber auch die Prüfstation 2 mit dem bereits genannten Photo-Ionisations-Detektor versehen sein.

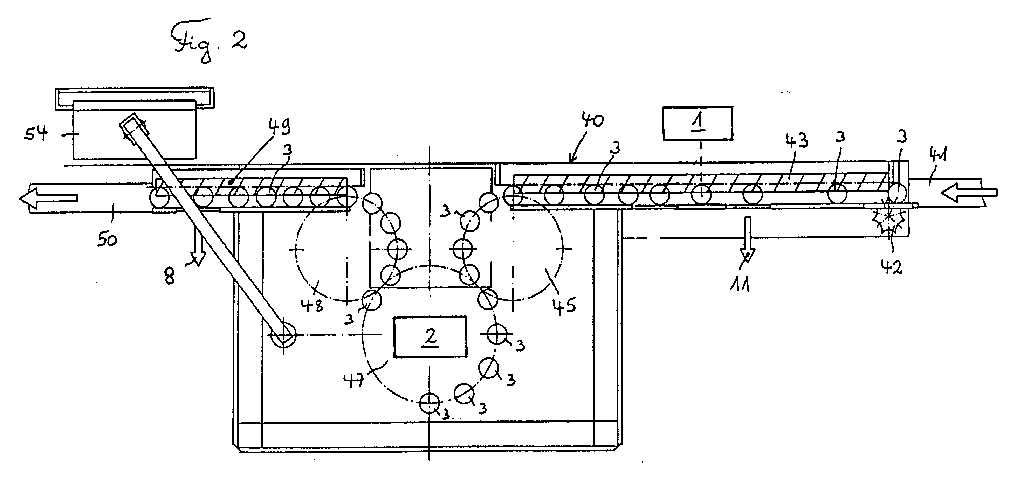

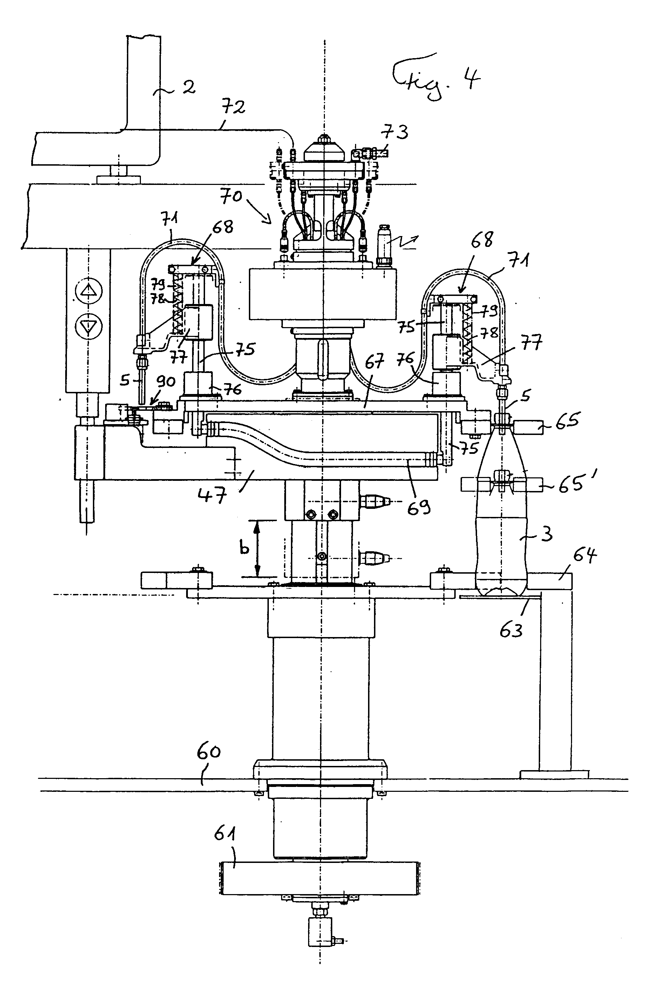

Figur 2 zeigt schematisch eine Draufsicht auf eine nach dem Verfahren arbeitende Vorrichtung 40 zum Prüfen von Flaschen. Zurückkehrende Mehrwegflaschen werden ungeordnet auf einer Förderlinie 41 an die Vorrichtung abgegeben. Die Flaschen werden stehend an die Vorrichtung abgegeben und sind in der Regel offen, d.h. nicht mehr mit einem Deckel versehen und noch nicht gewaschen. Mit einem Conveyor und entsprechendem Staudruck werden die Flaschen 3 einem Kontrollstern 42 zugeführt, der auch den Linienstop bildet, und an eine Förderschnekke 43 abgegeben, welche die Flaschen in regelmässig beabstandeter Reihenfolge stehend weiter fördert. Entlang dieser Förderstrecke 43 können noch vor der Prüfstation 1 mehrere Prüfanordnungen vorgesehen sein, welche nachfolgend anhand der Figur 3 noch im Detail erläutert werden. Insbesondere können die Flaschen auf korrekte Höhe und auf das Vorhandensein eines Deckels oder eines sonstigen Verschlusses und einer Restmenge von Flüssigkeit geprüft werden. Nicht geeignete Flaschen können durch einen Auswerfer 11 aus der Förderschnecke ausgeworfen werden. Die Flaschen gelangen dann zu der Prüfstation 1, dem Photo-Ionisations-Detektor, und werden, falls als verschmutzt erkannt, in der Steuereinrichtung entsprechend vorgemerkt bzw. bei der alternativen Ausführung aus dem Förderweg ausgeschieden. Die derart vorgeprüften Flaschen 3 gelangen von der Schnecke 43 auf ein Einlauf-Karussell 45. Von diesem Einlauf-Karussel 45 werden die Flaschen an ein Hauptkarussell 47 abgegeben. Während der Aufenthaltsdauer der Flaschen im Hauptkarussell 47 erfolgt die Prüfung in der Prüfstation 2 z.B. als massenspektrometrische Prüfung. In der Figur 3 sind nur schematisch vier Flaschen 3 dargestellt. In der Realität wird das Hauptkarussell aber eine grössere Anzahl zu prüfender Flaschen aufnehmen können, z.B. 16 Flaschen, welche von einem oberhalb des Hauptkarussells angeordneten Massenspektrometer geprüft werden. Nach der Prüfung gelangen die geprüften Flaschen über ein Auslauf-Karussell 48 auf eine Auslaufschnecke 49. Bei dieser Auslaufschnecke ist ein weiterer Auswerfer 53 angeordnet, welcher diejenigen Flaschen auswirft, welche vom Massenspektrometer als verunreinigt erkannt worden sind. Das Auswerfen kann dabei auf verschiedene, bekannte Weisen erfolgen, z.B. durch einen Druckluftstrahl oder durch einen elektromechanisch betätigten Auswurf-Stössel. Bevorzugt ist aber ein "sanftes" Ausleitsystem, welches die auszuscheidenden Flaschen stehend einer anderen Förderstrecke zuführt. Dadurch wird das Umkippen von eventuell schädliche Flüssigkeitsmengen enthaltenden Flaschen vermieden. Diese Flaschen werden stehend an eine Entsorgungsstelle gefördert. Nach der Förderschnecke 49 werden die nicht kontaminierten Flaschen an eine Förderlinie 50 abgegeben, mittels welcher sie an eine Wasch-Station und nachfolgend an eine Befüll-Station abgegeben werden. An einem Ausleger ist ein Bedienschrank 54 angeordnet. Die Steuerung der gesamten Vorrichtung kann separat untergebracht sein.

Figur 3 zeigt schematisch die in der Vorrichtung gemäss Figur 3 vorgenommenen Prüfschritte. Die auf der Förderlinie ankommenden Flaschen gelangen zum Kontrollstern 42, wobei liegende Flaschen durch den Stern 42 einen Linienstop herbeiführen. In der Einlaufschnecke findet nachfolgend mittels zweier Lichtschranken eine Kontrolle der Höhe jeder einzelnen Flasche statt. Zu grosse oder zu kleine Flaschen werden ausgeschieden. Als nächstes erfolgt mittels eines Ultraschallsensors eine Kontrolle, ob bei jeder Flasche der Flaschendeckel entfernt ist. Flaschen mit Flaschendeckel oder sonstigem Verschluss werden ausgeschieden. Mittels eines Gewichtssensors erfolgt eine Kontrolle, ob sich noch eine grössere Menge Restflüssigkeit in der Flasche befindet. Ist dies der Fall, so wird die Flasche ausgeschieden.

Die nicht ausgeschiedenen Flaschen gelangen, wie bereits beschrieben, zu der Prüfstation 1.

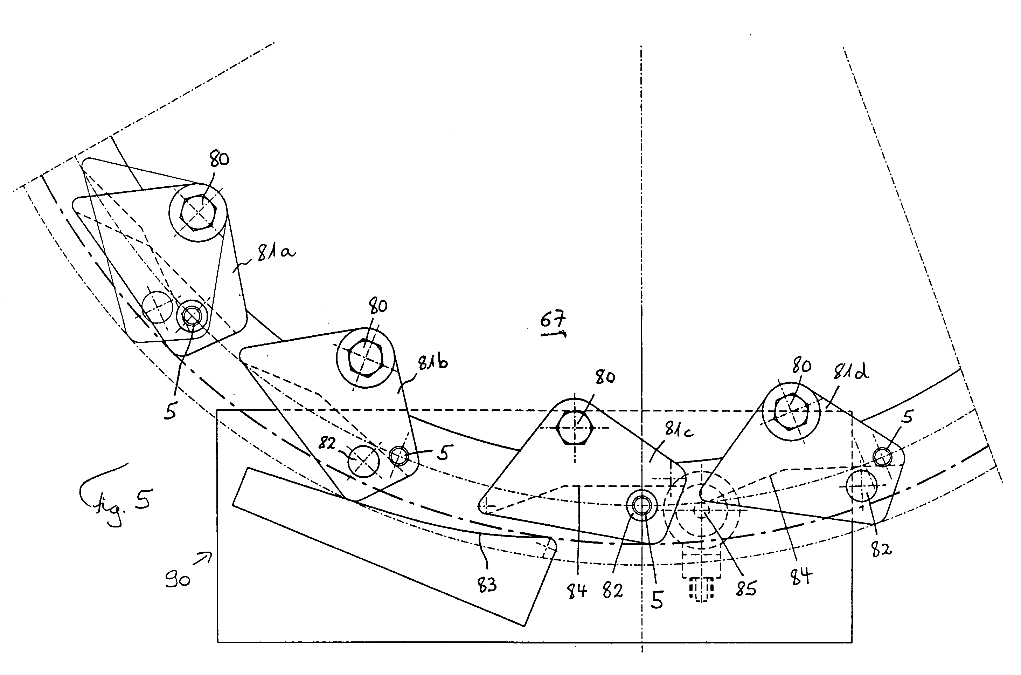

Figur 4 zeigt zeigt das Hauptkarussell 47 von Figur 2, wobei nur zwei Flaschenhalterungen und nur eine Flasche 3 dargestellt sind. Das Karussell ist an einem feststehenden Maschinentisch 60 angeordnet und wird über ein Antriebsrad 61 von einem nicht dargestellten Motor in Drehung versetzt. Die Flaschen 3 bewegen sich oberhalb eines maschinenfesten Bodenleitbleches 63 und sind im Flaschenbodenbereich jeweils in einer um die zentrale Achse des Karussells umlaufenden Aussenführung 64 gehalten und im Flaschenhalsbereich in einer umlaufenden Aussenführung 65. Der Abstand zwischen den Führungen 64 und 65 kann durch eine Verschiebung des oberen Karussellteils gegenüber dem unteren Karussellteil eingestellt werden, was durch den Pfeil b und durch die angedeutete Führung 65' dargestellt wird, welche die Führung 65 in ihrer unteren Position für eine Flasche mit geringerer Höhe zeigt. An dem sich drehenden oberen Karusselltisch 67 ist neben den genannten Aussenführungen 65 für jede Flasche jeweils eine Doppelhubeinheit 68 angeordnet, welche jeweils das Gasentnahmerohr 5 für jede Flasche trägt. Bei einem Karussell mit 16 Flaschenpositionen sind also 16 solche Doppelhubeinheiten 68 vorgesehen, deren Funktion weiter unten erläutert wird. Von jeder Doppelhubeinheit 68 wird ein Gasproben-Entnahmerohr 5 getragen, welches über einen flexiblen Schlauch 71 mit einem zentralen Ventilkopf 70 verbunden ist. Im zentralen Ventilkopf erfolgt die Verteilung der aus den Flaschen 3 abgesaugten Luft auf eine einzige Leitung 72, welche zu dem nur angedeuteten Massenspektrometer 2 führt. Dabei wird vorzugsweise während des grössten Teils des Umlaufes jeder Flasche 3 im Karussell Gas aus der Flasche 3 abgesaugt, was mittels einer zentralen Vakuumpumpe erfolgt (nicht dargestellt), welche am Anschluss 73 des Ventilkopfes 70 angeschlossen ist. Im Ventilkopf 70 erfolgt dann jeweils kurzzeitig die Verbindung eines der Gasproben-Entnahmerohre 5 mit dem Massenspektrometer 2, während in der anderen Zeit die übrigen Gasproben durch den Anschluss 73 zur Pumpe abgeführt werden. Auf diese Weise wird der Einfluss des langen Förderweges vom Entnahmerohr 5 bis zum Ventilkopf 70 auf die Messzeit eliminiert.

Bei der in Figur 4 gezeigten Vorrichtung ist nun oberhalb jeder Flasche 3 ein Verschluss vorgesehen, der das Eintauchen des Gasproben-Entnahmerohres 5 in die Flasche 3 verhindern kann. Dieser Verschluss 90 ist in Figur 4 nur unterhalb des linken Entnahmerohrs 5 angedeutet und beim rechten Entnahmerohr nicht dargestellt, obschon ein solcher Verschluss auch dort vorzusehen ist. Die Ausführung des Verschlusses wird anhand der Figur 5 näher erläutert werden. Die Doppelhubeinheiten 68 weisen jeweils einen Hubstössel 75 auf, der in einer Führung 76 verschieblich gehalten ist. Mit seinem unteren Ende greift jeder Hubstössel an einer Hubkurve 69 an, welche gegenüber dem Karussellteil 67 feststeht. Die Hubkurve 69 bewirkt damit beim Umlaufen des Karussellteils 67 eine Verschiebung des Stössels 75 in vertikaler Richtung. Der in der rechten Hälfte der Figur 4 dargestellte Stössel 75 befindet sich dabei in seiner unteren Endlage, bei welcher das Gasprobe-Entnahmerohr 5 in die Flasche 3 eintaucht und der in der linken Hälfte von Figur 4 gezeigte Stössel 75 befindet sich in seiner oberen Endlage, bei welcher das Gasprobe-Entnahmerohr 5 nicht mehr in die Flasche eintaucht und sich oberhalb des Verschlusses 90 befindet. Die Gasproben-Entnahmerohre 5 sind dabei an einem Schlitten 77 angeordnet, welcher entlang des Stössels verschieblich ist. Der Schlitten 77 wird jedoch im Normalfall durch eine Feder 78 in eine Endlage seines Verfahrweges auf dem Stössel 75 vorgespannt. Wenn das Gasproben-Entnahmerohr 5 ohne auf einen Widerstand zu treffen in die Flasche eintauchen kann, so ergibt sich keine Verschiebung des Schlittens 77 gegenüber dem Stössel 75, sondern der Schlitten 77 folgt jeweils der durch die Hubkurbe 69 bewirkten Stösselbewegung 75 und taucht in die Flasche 3 ein, beziehungsweise zieht sich wieder aus dieser zurück. Ist jedoch der Verschluss 90 geschlossen, wie nachfolgend noch dargestellt werden wird, so trifft das Gasproben-Entnahmerohr 5 beim Absenken des Stössels 75 auf den Verschluss 90 auf und kann deshalb nicht in die Flasche 3 abgesenkt werden. In diesem Fall bewegt sich zwar der Stössel 75 weiter nach unten, um die Absenkbewegung durchzuführen, doch das Gasprobe-Entnahmerohr 5 und der Schlitten 77 führen diese Abwärtsbewegung nicht aus. Der Stössel 75 verschiebt sich dabei relativ zu dem feststehenden Schlitten 77, wobei die Feder 78 gespannt wird. Das Gasproben-Entnahmerohr 5 bleibt während des Umlaufes dieser vom Verschluss 90 gesperrten Flasche oberhalb dieser Flasche blockiert und saugt auch in dieser Lage keine Luft aus der Flasche 3 an, da der Verschluss 90 dies verhindert. Beim Hochfahren des Stössels 75 fährt dieser zunächst relativ zum weiter feststehenden Schlitten 77 in die Höhe, bis die normale Lage des Schlittens 77 auf dem Stössel 75, welche durch die von der Feder 78 umgebene Anschlagstange 79 bestimmt ist, erreicht ist. Sobald beim Hochfahren des Stössels 75 durch die Feder 78 die Normallage von Stössel und Schlitten zueinander wieder hergestellt worden ist, bewegen sich diese Elemente wieder synchron miteinander, sofern nicht bei der nächsten Flasche, in welche das Gasproben-Entnahmerohr 5 eintauchen muss, erneut der Verschluss 90 ein solches Eintauchen verhindert.

Figur 5 zeigt einen Teil des Verschlusses 90. Dabei ist das sich in Pfeilrichtung drehende Karussellteil 67 von oben ersichtlich. An diesem ist für jede Flaschenposition eine um eine Drehachse 80 drehbare Verschlussklappe 81 vorgesehen. Bei 16 Flaschenpositionen sind also 16 solche Klappen 81 vorgesehen. Die Klappen 82 weisen eine Durchtrittsöffnung 82 für das Gasproben-Entnahmerohr 5 auf. Je nach Schwenklage der jeweiligen Klappe 81 (Durchtrittsposition oder Verschliessposition) kann nun das oberhalb der Klappe 81 befindliche Rohr 5 bei dessen Absenkung in die Flasche 3 eintreten oder nicht. Zur Einstellung der Klappen 81 werden diese bei jedem Umlauf zunächst durch eine Rückstellkurve 83 in die Offenposition gebracht, wie dies bei der Klappe 81 c ersichtlich ist. Danach erfolgt durch einen von der Steuereinheit angesteuerten elektromagnetisch betätigten Stössel 85, der bei Betätigung an eine Steuerkante 84 an der Unterseite jeder Klappe 81 angreifen kann, eine individuelle Einstellung der jeweiligen Klappe 81 in die Schliessposition, was bei der Klappe 81 d gezeigt ist, wenn diese aufgrund des Prüfresultates der Prüfstation 1 für die der Klappe auf ihrem Umlauf zugeordnete Flasche 3 nötig ist, d.h. wenn es sich bei dieser Flasche um eine als verschmutzt erkannte Flasche handelt. Die Schliessposition der Klappe, wie bei Klappe 81 d gezeigt, verhindert das Eintauchen des Proben-Entnahmerohres 5 in die Flasche während des ganzen Umlaufes der Flasche auf dem Karussell. Nachdem diese verschmutzte Flasche das Karussell verlassen hat, gelangt die Klappe wieder zu der Rückstellkurve 83, wo sie in Offenposition gebracht wird und nachfolgend in dieser Position verbleibt, sofern die nächste Flasche eine saubere Flasche ist, bzw. die Klappe erneut geschlossen wird, wenn die nächste, der Klappe zugeordnete Flasche 3 wieder eine verschmutzte Flasche ist.

Anstelle der gezeigten Zwangssteuerung der Entnahmerohre 5 durch die Karussellbewegung kann auch jedes Rohr 5 mit einem individuell steuerbaren Antrieb versehen werden. In diesem Fall kann auf den Verschlussmechanismus 90 verzichtet werden.

Das Gasproben-Entnahmerohr 5 wird dann jeweils durch die Steuereinheit gesteuert und von seinem individuellem Antrieb angetrieben in die Flasche 3 eingefahren oder nicht, je nachdem ob eine zu prüfende oder eine verschmutzte, nicht zu prüfende Flasche in das Karussell gelangt. Die gezeigte Lösung mit dem Verschlussmechanismus 90 bietet dem gegenüber aber insbesondere den Vorteil, das die jeweilige Klappe 81 eine Sperre für das Ansaugen von Gas aus der Flasche 3 bildet, das, wenn auch in geringem Masse, auch bei oberhalb der Flasche 5 in nicht eingefahrener Position des Rohres 5 erfolgen kann, wenn keine solche Sperre vorhanden ist.

## Patentansprüche

1. Verfahren zum Prüfen von zur erneuten Befüllung rücklaufenden Mehrwegflaschen, insbesondere PET-Flaschen, auf das Vorhandensein von Verunreinigungen, durch Prüfung von jeweils jeder Flasche entnommenem Gas, wobei die Flaschen entlang eines Förderweges transportiert werden, dadurch gekennzeichnet, dass die Prüfung jeder Flasche durch mindestens zwei entlang des Förderweges nacheinander angeordnete Prüfstationen erfolgt, wobei die erste Prüfstation eine untere Ansprechschwelle für Verunreinigungen aufweist, welche ein Mehrfaches der unteren Erkennungsschwelle für Verunreinigungen der zweiten Prüfstation beträgt, und dass bei Flaschen, auf deren Verunreinigung die erste Prüfstation angesprochen hat, bei deren Durchlauf durch die zweite Prüfstation die zweite Prüfstation deaktiviert wird.

2. Verfahren nach Anspruch 1, dadurch gekennzeichnet, dass die zweite Prüfstation dadurch deaktiviert wird, dass das Eintauchen eines Gasentnahmeorgans in die zu prüfende Flasche verhindert wird.

3. Verfahren nach Anspruch 2, dadurch gekennzeichnet, dass in der zweiten Prüfstation jeweils bei denjenigen Flaschen, auf deren Verunreinigung die erste Prüfstation angesprochen hat, ein Verschlusselement über der Flaschenöffnung positioniert wird.

4. Verfahren zum Prüfen von zur erneuten Befüllung rücklaufenden Mehrwegflaschen, insbesondere PET-Flaschen, auf das Vorhandensein von Verunreinigungen, durch Prüfung von jeweils jeder Flasche entnommenem Gas, wobei die Flaschen entlang eines Förderweges transportiert werden, dadurch gekennzeichnet, dass die Prüfung jeder Flasche durch mindestens zwei entlang des Förderweges nacheinander angeordneten Prüfstationen erfolgt, wobei die erste Prüfstation eine untere Ansprechschwelle für Verunreinigungen aufweist, welche ein Mehrfaches der unteren Erkennungsschwelle für Verunrenigungen der zweiten Prüfstation beträgt, und dass Flaschen, auf deren Verunrenigung die erste Prüfstation angesprochen hat, vor Erreichen der zweiten Prüfstation aus dem Förderweg ausgeschieden werden.

5. Verfahren nach einem der Ansprüche 1 bis 4, dadurch gekennzeichnet, dass die erste Prüfstation der Flasche eine Gasprobe entnimmt und diese durch Photo-Ionisations-Detektion prüft, und dass die zweite Prüfstation der Flasche eine weitere Gasprobe entnimmt und diese durch Photo-Ionisations-Detektion prüft.

6. Verfahren nach einem der Ansprüche 1 bis 4, dadurch gekennzeichnet, dass die erste Prüfstation der Flasche eine Gasprobe entnimmt und diese durch Photo-Ionisations-Detektion prüft, und dass die zweite Prüfstation der Flasche eine weitere Gasprobe entnimmt und diese durch massenspektrometrische Prüfung prüft.

7. Verfahren nach einem der Ansprüche 1 bis 6, dadurch gekennzeichnet, dass vor der ersten Prüfstation jede Flasche eine Höhenkontrolle durchläuft und auf das Vorhandensein eines Flaschendeckels und von Restflüssigkeit in der Flasche geprüft wird.

8. Vorrichtung zur Durchführung des Verfahrens nach einem der Ansprüche 1,2,3,5,6, gekennzeichnet durch eine Fördereinrichtung für rücklaufende Flaschen, eine erste an der Fördereinrichtung angeordnete Prüfstation, welche eine erste Gasproben-Entnahmeeinheit und eine erste Prüfeinheit umfasst, eine zweite, an der Fördereinrichtung in Flaschenförderrichtung hinter der ersten Prüfstation angeordnete Prüfstation mit einer zweiten Gasproben-Entnahmeeinheit und einer zweiten Prüfeinheit, und durch eine Steuereinheit, durch welche die zweite Gasproben-Entnahmeeinheit in Abhängigkeit vom Ausgangssignal der ersten Prüfeinheit deaktivierbar ist.

9. Vorrichtung nach Anspruch 8, dadurch gekennzeichnet, dass die Fördereinrichtung im Bereich der zweiten Prüfstation als Karussell ausgestaltet ist, wobei jeder zu prüfenden Flaschen jeweils eine in das Flascheninnere absenkbare Gasproben-Entnahmeeinheit zugeordnet ist.

10. Vorrichtung nach Anspruch 9, dadurch gekennzeichnet, dass die absenkbare Gasproben-Entnahmeeinheit jeweils ein Gasprobenrohr aufweist, welches jeweils mittels einer ersten Hubeinheit in die Entnahmestellung absenkbar ist, und dass die Absenkung jeweils durch eine Verschlussklappe blockierbar ist, wobei bei blockierter Absenkung das abzusenkende Gasprobenrohr von der Absenkbewegung der ersten Hubeinheit durch eine zweite Hubeinheit entkoppelt ist.

11. Vorrichtung nach Anspruch 10, dadurch gekennzeichnet, dass die Verschlussklappen jeweils um eine Achse schwenkbar an einem drehbaren Karussellteil angeordnet sind und von einer demgegenüber feststehenden Rückstellkurve in die Offenstellung schwenkbar bzw. von einem von der Steuereinheit betätigbaren, demgegenüber feststehenden Schaltnocken individuell in die Schliessstellung schwenkbar sind.

12. Vorrichtung zur Durchführung des Verfahrens nach Anspruch 4, gekennzeichnet durch eine Fördereinrichtung für rücklaufende Flaschen und durch eine erste an der Fördereinrichtung angeordnete Prüfstation, welche eine Gasproben-Entnahmeeinheit und einen Photo-Ionisations-Detektor umfasst und eine zweite, an der Fördereinrichtung in Flaschenförderrichtung hinter der ersten Prüfstation angeordnete Prüfstation mit einer weiteren Gasproben-Entnahmeeinheit und einer Prüfeinheit, sowie durch eine zwischen den Gasproben-Entnahmeeinheiten angeordnete Anordnung zur Ausscheidung (7) von Flaschen aus dem Förderweg.

13. Vorrichtung nach Anspruch 12, dadurch gekennzeichnet, dass die Prüfeinheit einen Photo-Ionisations-Detektor aufweist.

14. Vorrichtung nach Anspruch 12, dadurch gekennzeichnet, dass die Prüfeinheit ein Massenspektrometer aufweist.

## Claims

1. A procedure for testing returnable bottles being returned for refilling, particularly PET bottles, for the presence of contaminants, by testing gas withdrawn in each case from each bottle, wherein the bottles are transported along a conveying path, characterised in that testing of each bottle is performed by at least two test stations disposed in series along the conveying path, wherein the first test station has a lower response threshold for contaminants which is a multiple of the lower identification threshold for contaminants of the second test station, and that when there are bottles for which the first test station has responded to their contamination the second test station is deactivated when they pass through the second test station.

2. A procedure according to claim 1, characterised in that the second test station is deactivated in that the immersion of a gas withdrawal element in the bottle to be tested is prevented.

3. A procedure according to claim 2, characterised in that in the second test station a closure element is positioned over the bottle opening of each of those bottles for which the first test station has responded to their contamination.

4. A procedure for testing returnable bottles being returned for refilling, particularly PET bottles, for the presence of contaminants, by testing gas withdrawn in each case from each bottle, wherein the bottles are transported along a conveying path, characterised in that testing of each bottle is performed by at least two test stations disposed in series along the conveying path, wherein the first test station has a lower response threshold for contaminants which is a multiple of the lower identification threshold for contaminants of the second test station, and that bottles for which the first test station has responded to their contamination are removed from the conveying path before reaching the second test station.

5. A procedure according to any one of claims 1 to 4, characterised in that the first test station withdraws a gas sample from the bottle and tests this by photo-ionisation detection, and that the second test station withdraws a further gas sample and tests this by photo-ionisation detection.

6. A procedure according to any one of claims 1 to 4, characterised in that the first test station withdraws a gas sample from the bottle and tests this by photo-ionisation detection, and that the second test station withdraws a further gas sample and tests this by mass spectrometric testing.

7. A procedure according to any one of claims 1 to 6, characterised in that before the first test station each bottle passes through a height check and is tested for the presence of a bottle cap and for residual liquid in the bottle.

8. An apparatus for carrying out the procedure according to any one of claims 1, 2, 3, 5 or 6, characterised by a conveyor device for the returning bottles, a first test station disposed on the conveyor device and which comprises a first gas sample withdrawal unit and a first testing unit, a second test station disposed on the conveyor device behind the first test station in the bottle conveying direction and having a second gas sample withdrawal unit and a second testing unit, and by a control unit by means of which the second gas sample withdrawal unit can be deactivated depending on the output signal of the first testing unit.

9. An apparatus according to claim 8, characterised in that the conveyor device is arranged as a carousel in the region of the second test station, wherein a gas sample withdrawal unit which can be lowered into the inside of the bottle in each case is associated with each of the bottles to be tested.

10. An apparatus according to claim 9, characterised in that each lowerable gas sample withdrawal unit comprises a gas sample tube which can be lowered into the withdrawal position each time by means of a first lifting unit, and that the lowering operation can be blocked each time by a closure flap, wherein when lowering is blocked the gas sample tube to be lowered is uncoupled from the lowering movement of the first lifting unit by a second lifting unit.

11. An apparatus according to claim 10, characterised in that the closure flaps are each disposed on a rotatable carousel part so that they can be swung about an axis, and can be swung individually into the open position by a restoring cam which is disposed fixed in relation to the rotatable carousel part or can be swung individually into the closed position by a control cam which can be operated by the control unit and which is disposed fixed in relation to the rotatable carousel part.

12. An apparatus for carrying out the procedure according to claim 4, characterised by a conveyor device for the returning bottles, a first test station disposed on the conveyor device and which comprises a first gas sample withdrawal unit and a photo-ionisation detector, and a second test station disposed on the conveyor device behind the first test station in the bottle conveying direction and having a second gas sample withdrawal unit and a testing unit, and by an arrangement disposed between the gas sample withdrawal units for the removal (7) of bottles from the conveying path.

13. An apparatus according to claim 12, characterised in that the testing unit comprises a photo-ionisation detector.

14. An apparatus according to claim 12, characterised in that the testing unit comprises a mass spectrometer.

## Revendications

1. Procédé pour détecter la présence de salissures dans des bouteilles récupérables recyclées en vue d'un nouveau remplissage, en particulier de bouteilles en PET, par test de gaz prélevé dans chaque bouteille, les bouteilles étant transportées le long d'un trajet d'avance, caractérisé en ce que le test de chaque bouteille est effectué au moyen d'au moins deux postes de test disposés l'un derrière l'autre le long du trajet d'avance, le premier poste de test possédant un seuil de réponse pour les salissures qui est un multiple du seuil de reconnaissance de salissures du deuxième poste de test, et en ce que, dans le cas de bouteilles dont les salissures ont provoqué la réponse du premier poste de test, le deuxième poste de test est désactivé lors du passage des bouteilles dans ce second poste.

2. Procédé suivant la revendication 1, caractérisé en ce que le deuxième poste de test est désactivé en empêchant l'introduction d'un organe de prélèvement de gaz dans la bouteille à tester.

3. Procédé suivant la revendication 2, caractérisé en ce que, dans le deuxième poste de test, pour chaque bouteille dont les salissures ont provoqué la réponse du premier poste de test, un élément d'obturation est positionné au-dessus du goulot de la bouteille.

4. Procédé pour détecter la présence de salissures dans des bouteilles récupérables recyclées en vue d'un nouveau remplissage, en particulier de bouteilles en PET, par test de gaz prélevé dans chaque bouteille, les bouteilles étant transportées le long d'un trajet d'avance, caractérisé en ce que le test de chaque bouteille est effectué au moyen d'au moins deux postes de test disposés l'un derrière l'autre le long du trajet d'avance, le premier poste de test possédant un seuil de réponse pour les salissures qui est un multiple du seuil de reconnaissance de salissures du deuxième poste de test, et en ce que les bouteilles dont les salissures ont provoqué la réponse du premier poste de test sont évacuées du trajet d'avance avant d'atteindre le deuxième poste de test.

5. Procédé suivant l'une des revendications 1 à 4, caractérisé en ce que le premier poste de test prélève dans les bouteilles un échantillon de gaz et teste celui-ci par détection par photo-ionisation, et en ce que le deuxième poste de test prélève dans les bouteilles un autre échantillon de gaz, et teste celui-ci par détection par photo-ionisation.

6. Procédé suivant l'une des revendications 1 à 4, caractérisé en ce que le premier poste de test prélève dans les bouteilles un échantillon de gaz et teste celui-ci par détection par photo-ionisation, et en ce que le deuxième poste de test prélève dans les bouteilles un autre échantillon de gaz et teste celui-ci par spectrométrie de masse.

7. Procédé suivant l'une des revendications 1 à 6, caractérisé en ce qu'on fait subir à chaque bouteille, avant le premier poste de test, un contrôle de hauteur, et on détecte la présence d'un bouchon ou de liquide résiduel.

8. Dispositif pour la mise en oeuvre du procédé suivant l'une quelconque des revendications 1, 2, 3, 5 et 6, caractérisé par un convoyeur pour des bouteilles recyclés, un premier poste de test disposé le long du convoyeur et comportant une première unité de prélèvement d'échantillons gazeux ainsi qu'une première unité de détection, un deuxième poste de test disposé le long du convoyeur derrière le premier poste de test en considérant le sens d'avance des bouteilles, et comprenant une deuxième unité de prélèvement d'échantillons gazeux ainsi qu'une deuxième unité de détection, et par une unité de commande au moyen de laquelle la deuxième unité de prélèvement d'échantillons de gaz peut être désactivée en fonction du signal de sortie de la première unité de détection.

9. Dispositif suivant la revendication 8, caractérisé en ce que, dans la région du deuxième poste de test, le convoyeur est réalisé sous la forme d'un carrousel, une unité de prélèvement d'échantillons gazeux, qui peut être abaissée à l'intérieur des bouteilles, étant associée à chaque bouteille à tester.

10. Dispositif suivant la revendication 9, caractérisé en ce que chaque unité de prélèvement d'échantillons gazeux pouvant être abaissée comprend un tube d'échantillonnage de gaz qui peut être abaissé en position de prélèvement au moyen d'une première unité respective de déplacement vertical, et en ce que l'abaissement de chaque tube peut être bloqué par un volet d'obturation, le tube d'échantillonnage étant désaccouplé du mouvement de descente de la première unité de déplacement vertical par une seconde unité de déplacement vertical lorsque son mouvement vers le bas est bloqué.

11. Dispositif suivant la revendication 10, caractérisé en ce que chaque volet d'obturation est monté pivotant autour d'un axe sur une partie rotative du carrousel, et peut être amené par pivotement en position d'ouverture par une came de rappel fixe et être amené individuellement en position de fermeture par une came de manoeuvre fixe actionnable par l'unité de commande.

12. Dispositif pour la mise en oeuvre du procédé suivant la revendication 4, caractérisé par un convoyeur pour des bouteilles recyclées et par un premier poste de test disposé le long du convoyeur et comprenant une unité de prélèvement d'échantillons gazeux et un détecteur par photo-ionisation, par un deuxième poste de test disposé le long du convoyeur derrière le premier poste de test en considérant le sens d'avance des bouteilles, ce deuxième poste comprenant une autre unité de prélèvement d'échantillons gazeux et une unité de détection, ainsi que par un dispositif (7) d'évacuation des bouteilles du trajet d'avance, disposé entre les unités de prélèvement d'échantillons gazeux.

13. Dispositif suivant la revendication 12, caractérisé en ce que l'unité de détection comprend un détecteur par photo-ionisation.

14. Dispositif suivant la revendication 12, caractérisé en ce que l'unité de détection comprend un spectromètre de masse.
